# EUROPEAN PATENT SPECIFICATION

(11) **EP 2 021 794 B1**
(45) Date of publication and mention of the grant of the patent: **16.06.2010**
(21) Application number: 07725293.0
(22) Date of filing: 16.05.2007
(51) Int. Cl.: G01N 33/53, C12Q 1/68

(54) **USE OF PROTEIN S100A 12 AS A MARKER FOR COLORECTAL CANCER**
VERWENDUNG DES PROTEINS S100A 12 ALS MARKER FÜR KOLOREKTALKARZINOM
UTILISATION DE LA PROTÉINE S100A 12 EN TANT QUE MARQUEUR DU CANCER DU CÔLON ET DU RECTUM

(30) Priority: 19.05.2006 EP 06010439
(43) Date of publication of application: 11.02.2009
(73) Proprietor: Roche Diagnostics GmbH, 68305 Mannheim (DE); F.HOFFMANN-LA ROCHE AG, 4070 Basel (CH)
(72) Inventor: WILD, Norbert, 82538 Geretsried (DE); ANDRES, Herbert, 82377 Penzberg (DE); GARCZAREK, Ursula, 83671 Benediktbeuern (DE); GEISTANGER, Andrea, 81477 Muenchen (DE); HAGMANN, Marie-Luise, 82377 Penzberg (DE); KARL, Johann, 82380 Peissenberg (DE); KRAUSE, Friedemann, 82377 Penzberg (DE); PFEFFER, Michael, 82377 Penzberg (DE); ROLLINGER, Wolfgang, 82398 Polling (DE); TACKE, Michael, 80689 Muenchen (DE); THIEROLF, Michael, 88400 Biberach (DE)
(86) International application number: PCT/EP2007/004378
(87) International publication number: WO 2007/134779

(56) References cited:
- WO-A-2005/054508
- WO-A-2006/012588
- WO-A2-2004/079368
- US-A1- 2004 157 278
- SIEG A ET AL: "DETECTION OF COLORECTAL NEOPLASMS BY THE HIGHLY SENSITIVE HEMOGLOBIN-HAPTOGLOBIN COMPLEX IN FECES" INTERNATIONAL JOURNAL OF COLORECTAL DISEASE, SPRINGER VERLAG, BERLIN, DE, vol. 14, no. 6, December 1999 (1999-12), pages 267-271, XP001182520 ISSN: 0179-1958
- GILBERT J A ET AL: "FECAL MARKER VARIABILITY IN COLORECTAL CANCER: CALPROTECTIN VERSUS HEMOGLOBIN" SCANDINAVIAN JOURNAL OF GASTROENTEROLOGY, vol. 31, 1996, pages 1001-1005, XP002053475 ISSN: 0036-5521
- STULÍK J ET AL: "The analysis of S100A9 and S100A8 expression in matched sets of macroscopically normal colon mucosa and colorectal carcinoma: the S100A9 and S100A8 positive cells underlie and invade tumor mass." ELECTROPHORESIS. 1999 APR-MAY, vol. 20, no. 4-5, April 1999 (1999-04), pages 1047-1054, XP002407823 ISSN: 0173-0835
- STULÍK J ET AL: "Overexpression of calcium-binding protein calgranulin B in colonic mucosal diseases." CLINICA CHIMICA ACTA; INTERNATIONAL JOURNAL OF CLINICAL CHEMISTRY. 8 SEP 1997, vol. 265, no. 1, 8 September 1997 (1997-09-08), pages 41-55, XP002403733 ISSN: 0009-8981

### SEQUENCE LISTING

<110> Roche Diagnostics GmbH
   F. Hoffmann-La Roche AG
<120> Use of S100A12 as a marker for colorectal cancer
<130> 23747 WO
<150> EP 06010439
   <151> 2006-05-19
<160> 1
<170> PatentIn version 3.2
<210> 1
   <211> 92
   <212> PRT
   <213> Homo sapiens
<400> 1
   Met Thr Lys Leu Glu Glu His Leu Glu Gly Ile Val Asn Ile Phe His
   1               5                   10                  15
   Gln Tyr Ser Val Arg Lys Gly His Phe Asp Thr Leu Ser Lys Gly Glu
               20                  25                  30
   Leu Lys Gln Leu Leu Thr Lys Glu Leu Ala Asn Thr Ile Lys Asn Ile
           35                  40                  45
   Lys Asp Lys Ala Val Ile Asp Glu Ile Phe Gln Gly Leu Asp Ala Asn
       50                  55                  60
   Gln Asp Glu Gln Val Asp Phe Gln Glu Phe Ile Ser Leu Val Ala Ile
   65                  70                  75                  80
   Ala Leu Lys Ala Ala His Tyr His Thr His Lys Glu
                   85                  90

## Description

The present invention relates to the diagnosis of colorectal cancer. It discloses the use of the protein S100A12 (=Calgranulin C) as a marker molecule in the diagnosis of colorectal cancer. Furthermore, it especially relates to a method for diagnosis of colorectal cancer from a stool sample, derived from an individual by measuring S100A12 in said sample. Measurement of S100A12 can, e.g., be used in the early detection or diagnosis of colorectal cancer.

Cancer remains a major public health challenge despite progress in detection and therapy. Amongst the various types of cancer, colorectal cancer (=CRC) is one of the most frequent cancers in the Western world.

The earlier cancer can be detected/diagnosed, the better is the overall survival rate. This is especially true for CRC. The prognosis in advanced stages of tumor is poor. More than one third of the patients will die from progressive disease within five years after diagnosis, corresponding to a survival rate of about 40% for five years. Current treatment is only curing a fraction of the patients and clearly has the best effect on those patients diagnosed in an early stage of disease.

With regard to CRC as a public health problem, it is essential that more effective screening and preventative measures for colorectal cancer be developed.

The earliest detection procedures available at present for colorectal cancer involve using tests for fecal blood or endoscopic procedures. However, significant tumor size must typically exist before fecal blood is detected. With regard to detection of CRC from a stool sample, the current state of the art is the guaiac-based fecal occult blood test.

In the recent years a tremendous amount of so-called colon specific or even so-called colorectal cancer specific genes has been reported. The vast majority of the corresponding research papers or patent applications are based on data obtained by analysis of RNA expression patterns in colon (cancer) tissue versus a different tissue or an adjacent normal tissue, respectively. Such approaches may be summarized as differential mRNA display techniques.

As an example for data available from mRNA-display techniques, WO 01/96390 shall be mentioned and discussed. This application describes and claims more than two hundred isolated polynucleotides and the corresponding polypeptides as such, as well as their use in the detection of CRC. However, it is general knowledge that differences on the level of mRNA are not mirrored by the level of the corresponding proteins. A protein encoded by a rare mRNA may be found in very high amounts and a protein encoded by an abundant mRNA may nonetheless be hard to detect and find at all. This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of CRC. Brünagel, G. et al., Cancer Research 62 (2002) 2437-2442, have identified seven nuclear matrix proteins which appear to be more abundant in CRC tissue as compared to adjacent normal tissue. No data from liquid and stool samples obtained from an individual are reported.

WO 02/078636 reports about nine colorectal cancer-associated spots as found by surface-enhanced laser desorption and ionization (SELDI). These spots are seen more frequently in sera obtained from patients with CRC as compared to sera obtained from healthy controls. However, the identity of the molecule(s) comprised in such spot, e.g., its (their sequence), is not known.

Despite the large and ever growing list of candidate protein markers in the field of CRC, to date clinical/diagnostic utility of these molecules is not known. In order to be of clinical utility a new diagnostic marker as a single marker should be at least as good as the best single marker known in the art. Or, a new marker should lead to a progress in diagnostic sensitivity and/or specificity either if used alone or in combination with one or more other markers, respectively. The diagnostic sensitivity and/or specificity of a test is often assessed by its receiver-operating characteristics, which will be described in detail below.

At present, for example, diagnostic blood tests based on the detection of carcinoembryonic antigen (CEA), a tumor-associated glycoprotein, are available to assist diagnosis in the field of CRC. CEA is increased in 95% of tissue samples obtained from patients with colorectal, gastric, and pancreatic cancers and in the majority of breast, lung, and head and neck carcinomas (Goldenberg, D.M. et al., J. Natl. Cancer Inst. (Bethesda) 57 (1976) 11-22). Elevated CEA levels have also been reported in patients with nonmalignant disease, and many patients with colorectal cancer have normal CEA levels in the serum, especially during the early stage of the disease (Carriquiry, L.A. and Pineyro, A., Dis. Colon Rectum 42 (1999) 921-929; Herrera, M.A. et al., Ann. Surg. 183 (1976) 5-9; Wanebo, H.J. et al., N. Engl. J. Med. 299 (1978) 448-451). The utility of CEA as measured from serum or plasma in detecting recurrences is reportedly controversial and has yet to be widely applied (Martell, R.E. et al., Int. J. Biol. Markers 13 (1998) 145-149; Moertel, C.G. et al., JAMA 270 (1993) 943-947).

In light of the available data, serum CEA determination possesses neither the sensitivity nor the specificity to enable its use as a screening test for colorectal cancer in the asymptomatic population (Reynoso, G. et al., JAMA 220 (1972) 361-365; Sturgeon, C., Clin. Chem. 48 (2002) 1151-1159).

Samples taken from stool have the advantage that their sampling is easily possible by non-invasive means.

As mentioned above, the guaiac test is currently most widely used as a screening assay for CRC from stool. The guaiac test, however, has both poor sensitivity as well as poor specificity. The sensitivity of the guaiac-based fecal occult blood tests is ∼26%, which means 74% of patients with malignant lesions will remain undetected (Ahlquist, D.A., Gastroenterol. Clin. North Am. 26 (1997) 41-55).

The visualization of precancerous and cancerous lesions represents the best approach to early detection, but colonoscopy is invasive with significant costs, risks, and complications (Silvis, S.E. et al., JAMA 235 (1976) 928-930; Geenen, J.E. et al., Am. J. Dig. Dis. 20 (1975) 231-235; Anderson, W.F. et al., J. Natl. Cancer Institute 94 (2002) 1126-1133).

The sensitivity and specificity of diagnostic alternatives to the guaiac test have been recently investigated by Sieg, A. et al., Int. J. Colorectal Dis. 14 (1999) 267-271. Especially the measurement of hemoglobin and of the hemoglobin-haptoglobin complex from stool specimen have been compared. It has been noted that the hemoglobin assay has an unsatisfactory sensitivity for the detection of colorectal neoplasms. Whereas cancer in its progressed carcinoma stage is detected with a sensitivity of about 87% the earlier tumor stages are not detected with a sufficient sensitivity. The hemoglobin-haptoglobin complex assay was more sensitive in the detection of earlier stages of CRC. This more sensitive detection was accompanied by a poor specificity. Since poor specificity, however, translates to a high number of unnecessary secondary investigations, like colonoscopy, an assay with a poor specificity also does not meet the requirements of a generally accepted screening assay.

Calprotectin has been described as an alternative biomarker for the detection of CRC from stool samples in US 5,455,160 and correspondingly in the scientific literature by Roseth, A.G., et al. (Scand. J. Gastroenterol. 27 (1992) 793-798; Scand. J. Gastroenterol. 28 (1993) 1073-1076). Although calprotectin is a marker of inflammatory diseases its potential as a marker for the detection of CRC from stool is documented by several publications (Johne, B., et al., Scand. J. Gastroenterol. 36 (2001) 291-296; Limburg, P.J., et al., Am. J. Gastroenterol. 98 (2003) 2299-2305; Hoff, G., et al., Gut 53 (2004) 1329-1333). While the sensitivity and specificity of calprotectin are comparable to the immunological hemoglobin assay, calprotectin appears to have some characteristics favorable for a diagnostic biomarker as compared to hemoglobin. It is evenly distributed in feces, it is stable at room temperature making mail delivery of the sample to the laboratory feasible and it shows no interference with food components or pharmaceutical compounds (Ton, H., et al., Clin. Chim. Acta 292 (2000) 41-54). However, elevated concentrations of calprotectin, the heterodimer of S100A8 and S100A9, were detected in stool samples from patients suffering from CRC, Crohn's disease or inflammatory bowel disease. These results are in agreement with the more general role of calprotectin in inflammation (Ryckman, C., et al., J. Immunol. 170 (2003) 3233-3242). Hence, the use of calprotectin in gastroenterology is not limited to the detection of CRC but extends to other disesases, especially inflammatory bowel disease as reviewed by Poullis, A., et al. (J. Gastroenterol. Hepatol. 18 (2003) 756-762).

In international patent application WO 2004/079368 it is mentioned that S100 A8 or S100A9, respectively, are up-regulated in part of the CRC-tissue samples investigated.

Sieg A., et al., International Journal of Colorectal Tissue Disease, 14(6), 1999, p 267-271, report on the detection of colorectal cancer by sensitive measurement of the hemoglobin/haptoglobin complex in fecal samples.

In US 2004/157278 it is disclosed that up-regulation of the gene coding for human islet regenerating protein 1 (Reg1α) and up-regulation of other genes like the one coding for tissue inhibitor of metalloproteases 1 (TIMP-1) is associated with colorectal cancer.

A further alternative method to the guaiac test for detection of CRC in stool has been published recently and consists in the detection of the colorectal cancer-specific antigen, "minichromosome maintenance protein 2" (MCM2) by immunohistochemistry in colonic cells shed into stool. Due to the small study size, conclusion on the diagnostic value for detection of colorectal cancer is preliminary. However, the test seems to have only limited sensitivity to detect right-sided colon cancer (Davies, R.J. et al., Lancet 359 (2002) 1917-1919).

Recently, an assay for detection of pyruvate kinase M2 isoenzyme (M2-PK) has been introduced into the market (Schebo Biotech, Gießen, Germany). A comparison of the guaiac assay to the immuno assays for hemoglobin and M2-PK has for example been performed by Vogel, T. et. al., Dtsch. Med. Wochenschr. 130 (2005) 872-877. They show that the immunological assays are superior to the guaiac test and that at comparable specificity the M2-PK assay is less sensitive in detecting CRC as compared to the hemoglobin assay. Yet, the authors conclude that the usefulness of both these stool based assays is still questionable.

The identification of an early CRC tumor marker that would allow reliable cancer detection or provide early prognostic information by non-invasive means from a stool specimen could lead to a diagnostic assay that would greatly aid in the diagnosis and in the management of this disease. Therefore, an urgent clinical need exists to improve the diagnosis of CRC, especially from stool. It is especially important to improve the early diagnosis of CRC, since for patients diagnosed early on chances of survival are much higher as compared to those diagnosed at a progressed stage of disease.

It was the task of the present invention to investigate whether a new marker can be identified which may aid in CRC diagnosis. Preferably such marker would be present in stool and allow for a non-invasive diagnosis.

Surprisingly, it has been found that the use of the protein S100A12 as a marker for CRC can at least partially overcome the problems known from the state of the art.

The present invention therefore relates to a method for the diagnosis of colorectal cancer comprising the steps of
a) providing a stool sample obtained from an individual,
b) contacting said sample with a specific binding agent for S100A12 under conditions appropriate for formation of a complex between said binding agent and S100A12
c) determining the amount of complex formed in step (b), and
d) correlating the amount of complex determined in (c) to the diagnosis of colorectal cancer.

As the skilled artisan will appreciate, any such measurement of S100A12 is made in vitro. The patient sample is discarded afterwards. The patient sample is solely used for the in vitro method of the invention and no material of the patient sample is transferred back into the patient's body. Neither measurement of S100A12 nor the assessment of CRC is performed on the human or animal body.

The in vitro diagnostic procedure according to the present invention is used to assess the absence, the presence or the relative concentration of S100A12 in a stool sample. The value measured for S100A12 will aid the clinician in assessing CRC, e.g., in his establishing a clinical diagnosis and/or in his decision for an appropriate treatment.

In a preferred embodiment the stool sample is processed to obtain a processed sample liquid which is more convenient to handle than a stool specimen. Such processed sample is then incubated with the specific binding agent for S100A12. The present invention therefore also relates to a method for the diagnosis of colorectal cancer comprising the steps of
a) providing a stool sample obtained from an individual,
b) processing said sample to obtain a processed liquid sample,
c) contacting said processed liquid sample with a specific binding agent for S100A12 under conditions appropriate for formation of a complex between said binding agent and S100A12, and
d) correlating the amount of complex formed in (c) to the diagnosis of colorectal cancer.

Another preferred embodiment of the invention is a method for the diagnosis of colorectal cancer comprising the steps of
a) processing a stool sample obtained from an individual to obtain a processed liquid sample,
b) contacting said processed liquid sample with a specific binding agent for S100A12 under conditions appropriate for formation of a complex between said binding agent and S100A12,
c) determining the amount of complex formed in step (b), and
d) correlating the amount of complex determined in (c) to the diagnosis of colorectal cancer.

In a further preferred embodiment the present invention discloses a method for the diagnosis of colorectal cancer comprising the steps of providing a stool sample obtained from an individual, contacting said sample with a specific binding agent for S100A12 under conditions appropriate for formation of a complex between said binding agent and S100A12, contacting said sample with a specific binding agent for a second marker selected from the group consisting of hemoglobin/haptoglobin complex, hemoglobin, tissue inhibitor of metalloproteases 1 (TIMP-1), and tumor M2 pyruvate kinase (M2-PK) under conditions appropriate for formation of a complex between said binding agent and the second marker, detection the amount of complex formed for S100A12 and the at least one second marker, and correlating the amount of complexes determined to the diagnosis of colorectal cancer. In further preferred embodiments the method according to the present invention is based on the determination and of S100A12 and of hemoglobin as the second marker, the determination of S100A12 and of the hemoglobin/haptoglobin complex as the second marker, the determination of S100A12 and of TIMP-1 as the second marker, and the determination of S100A12 and of M2-PK as the second marker, respectively. As obvious to the skilled artisan the measurement of S100A12 and the measurement of the at least one second marker can be made from the same aliquot of a stool sample or of a processed stool sample, respectively, or from different aliquots of a patient's stool sample or from different aliquots of a patient's processed stool sample, respectively.

In another preferred embodiment the stool sample is processed to retrieve colonocytes which are then smeared on a microscopic slide. Such processed sample is then incubated with the specific binding agent for S100A12. The present invention therefore also relates to a method for the diagnosis of colorectal cancer comprising the steps of
a) providing a stool sample obtained from an individual,
b) processing said sample to retrieve colonocytes,
c) contacting said processed sample with a specific binding agent for S100A12 under conditions appropriate for formation of a complex between said binding agent and S100A12, and
d) correlating the amount of complex formed in (c) to the diagnosis of colorectal cancer.

The protein S100A12 (=Calgranulin C) is characterized by the sequence given SEQ ID N0: 1.

S100A12 is also called CAAF1; CAGC; calcium binding protein in amniotic fluid; calgranulin related protein; CGRP; calcium binding protein in amniotic fluid 1; Calgranulin C; ENRAGE (extracellular newly identified RAGE binding protein); neutrophil S100 protein; S100 calcium binding protein A12. The protein encoded by this gene is a member of the S100 family of proteins containing 2 EF-hand calcium-binding motifs. S100 proteins are localized in the cytoplasm and/or nucleus of a wide range of cells, and involved in the regulation of a number of cellular processes such as cell cycle progression and differentiation. S100 genes include at least 13 members which are located as a cluster on chromosome 1q21. This protein is proposed to be involved in specific calcium-dependent signal transduction pathways and its regulatory effect on cytoskeletal components may modulate various neutrophil activities.

WO 2005/054508 relates to methods of gene expression profiling using pools of polynucleotide arrays and to the association of such profiles to histopathological features of colorectal cancer tissue. S100 A12 is comprised amongst the several hundred differentially expressed genes mentioned in this patent application. Detection of S100A12 on the protein level in CRC tissue, a body fluid or in a stool sample is not shown.

The physiologically relevant structure of S100A12 is a Ca ²⁺-binding homodimer. As S100A12 is also released from cells, extracellular functions have been described e.g., regulation of inflammatory processes (Foell, D. et al. Clin. Chim. Acta 344 (2004) 37-51). Together with S100A8 (= calgranulin A) and S100A9 (calgranulin B) S100A12 is expressed in granulocytes, where the calgranulin proteins constitute up to 50 % of the soluble cytosolic protein content. Upon acute inflammation S100A12 is released and it has been found in different diseases including irritable bowl diseases, Morbus Crohn, Kawasaki's disease or rheumatoid arthritis (Burmeister, G. and Gallacchi, G., Inflammopharmacology 3 (1995) 221-230; Foell, D. et al., Rheumatology 42 (2003) 1383-1389). When the signal cascade of the S100A12 response was investigated it was linked to a novel inflammatory axis via the receptor for advanced glycated endproducts (=RAGE). This receptor can be found in various tissue types including e.g., endothelium and cells of the immune system (Hofmann, M.A. et al., Cell 97 (1999) 889 - 901; Schmidt, A. M. et al., J. Clin. Invest. 108 (2001) 949-955). Besides being involved in the inflammatory response, the RAGE-pathway has also been described in wound healing, tumor outgrowth or systemic amyloidosis (Hofmann, M.A. et al., supra; Schmidt, A.M. et al., supra; Yan, S.S. et al., Nat. Med. 9 (2003) 287-293; Hofmann, M.A. et al., Genes Immun. 3 (2002) 123-135). Hence S100A12 might have a broad range of effects. However, no relation to tumor processes has been described to date.

In a preferred embodiment, the novel marker S100A12 is used in the screening for CRC. When used in patient monitoring the diagnostic method according to the present invention may help to assess tumor load, efficacy of treatment and tumor recurrence in the follow-up of patients. Increased levels of S100A12 are directly correlated to tumor burden. After chemotherapy a short term (few hours to 14 days) increase in S100A12 may serve as an indicator of tumor cell death. In the long term follow-up of patients after surgery and/or chemotherapy (from 3 months to 10 years) an increase of S100A12 can be used as an indicator for tumor recurrence in the colorectum.

In a preferred embodiment the diagnostic method according to the present invention is used for screening purposes. I.e., it is used to assess subjects without a prior diagnosis of CRC by measuring the level of S100A12 in a stool sample and correlating the level measured to the presence or absence of CRC.

As the skilled artisan will readily appreciate, in such screening care has to be taken that an appropriate amount of a stool sample is used. Preferably a defined amount of stool sample is used for the measurement of S100A12 comprised therein. Preferably the amount of S100A12 is expressed in terms of amount of S100A12 per amount of stool, i.e., the relative concentration of S100A12 is given.

The diagnostic method may not only be used for the screening of the general asymptomatic population but also in an alternative preferred embodiment for surveillance of high risk individuals. Such a high risk individuals preferably are selected from the group consisting of individuals with iron deficiency anemia, first grade relatives of CRC-patients, patients with a family history of CRC and patients with newly detected polyps or a history of gastrointestinal neoplasia.

For a screening assay not only the AUC value is relevant. A quite critical requirement in a screening setting is a good enough sensitivity at a high and clinically relevant level of specificity. High specificity is crucial because if this requirement would not be fulfilled, this would result in a high number of false positive results accompanied by unnecessary follow-up procedures and distress for the patients. In other preferred embodiments the level of specificity is set to 96%, 97%, 98% or 99%, respectively, as compared to CRC-negative individuals of the clinically relevant screening population.

As the skilled artisan will appreciate a cut off-value for S100A12 is established based on S100A12-values as measured in the stool sample derived from individuals of a healthy normal population. The clinically relevant normal population in a screening setting preferably consists of clinically healthy individuals in the age of 55 to 65 years. An S100A12-value in a stool sample above an established cut-off value may be considered indicative for CRC or may at least warrant further diagnostic examination of the respective individual.

Colorectal cancer most frequently progresses from adenomas (polyps) to malignant carcinomas.

The staging of cancer is the classification of the disease in terms of extent, progression, and severity. It groups cancer patients so that generalizations can be made about prognosis and the choice of therapy.

The different stages of CRC used to be classified according to Dukes' stages A to D. Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, sixth edition, 2002). Once the TNM status is determined the patients are grouped into disease stages that are denoted by Roman numerals ranging form I to IV with IV being the most advanced disease stage. TNM staging and UICC disease stages correspond to each other as shown in the following Table 1 taken from Sobin L.H. and Wittekind (eds.) *supra.*

**Table 1: Interrelation of TNM staging and UICC disease stages**

| **UICC disease stage** | **T staging** | **N staging** | **M staging** |
|---|---|---|---|
| Stage 0 | Tis | N0 | M0 |
| Stage I | T1, T2 | N0 | M0 |
| Stage IIA | T3 | N0 | M0 |
| Stage IIB | T4 | N0 | M0 |
| Stage IIIA | T1, T2 | N1 | M0 |
| Stage IIIB | T3, T4 | N1 | M0 |
| Stage IIIC | Any T | N2 | M0 |
| Stage IV | Any T | Any N | M1 |

What is especially important is, that early diagnosis of CRC translates to a much better prognosis. Malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

In the sense of the present invention early diagnosis of CRC refers to a diagnosis at a tumor stage where no metastases at all (neither proximal = N0, nor distal = M0) are present, i.e. the stages of, Tᵢₛ, N0, M0 or T1-4; N0; M0. Tᵢₛ denotes carcinoma *in situ.*

It is preferred, that CRC is diagnosed when it has not yet fully grown through the bowel wall and thus neither the visceral peritoneum is perforated nor other organs or structures are invaded, i.e., that diagnosis is made at any stage from Tᵢₛ; N0; M0 to T3; N0; M0 (=Tᵢₛ-3; N0; M0).

The diagnostic method according to the present invention is based on a stool sample which is derived from an individual. The stool sample is extracted and S100A12 is specifically measured from this processed stool sample by use of a specific binding agent.

A specific binding agent is, e.g., a receptor for S100A12, a lectin binding to S100A12, an aptamer to S100A12, or an antibody to S100A12. A specific binding agent has at least an affinity of 10⁷ l/mol for its corresponding target molecule. The specific binding agent preferably has an affinity of 10⁸ l/mol or even more preferred of 10⁹ l/mol for its target molecule. As the skilled artisan will appreciate the term specific is used to indicate that other biomolecules present in the sample do not significantly bind to with the binding agent specific for S100A12. Preferably, the level of binding to a biomolecule other than the target molecule results in a binding affinity which is only 10%, more preferably only 5% of the affinity of the target molecule or less. A most preferred specific binding agent will fulfill both the above minimum criteria for affinity as well as for specificity.

A specific binding agent preferably is an antibody reactive with S100A12. The term antibody refers to a polyclonal antibody, a monoclonal antibody, fragments of such antibodies, as well as genetic constructs comprising the binding domain of an antibody. Any antibody fragment retaining the above criteria of a specific binding agent can be used. Antibodies are generated by state of the art procedures, e.g., as described in Tijssen (Tijssen, P., Practice and theory of enzyme immunoassays 11 (1990) the whole book, especially pages 43-78, Elsevier, Amsterdam). In addition, the skilled artisan is well aware of methods based on immunosorbents that can be used for the specific isolation of antibodies. By these means the quality of polyclonal antibodies and hence their performance in immunoassays can be enhanced (Tijssen, P., *supra,* pages 108-115).

For the achievements as disclosed in the present invention polyclonal antibodies raised in rabbits have been used. However, clearly also polyclonal antibodies from different species , e.g. rats or guinea pigs, as well as monoclonal antibodies can also be used. Since monoclonal antibodies can be produced in any amount required with constant properties, they represent ideal tools in development of an assay for clinical routine. The generation and use of monoclonal antibodies to S100A12 in a method according to the present invention is yet another preferred embodiment.

As the skilled artisan will appreciate now, that S100A12 has been identified by measurement in an immunoassay as a marker which is useful in the diagnosis of CRC, alternative ways may be used to reach a result comparable to the achievements of the present invention. The marker protein S100A12 may be detected by any appropriate means and used as a marker of CRC. Such preferred appropriate means comprise the detection of the S100A12 polypeptide by an immuno assay procedure, by liquid chromatography, especially high performance liquid chromatography, by electrophoresis, especially SDS-PAGE combined with Western Blotting and by mass spectroscopy.

For measurement, the stool sample is obtained from an individual. An aliquot of the stool sample may be used directly. Preferably an aliquot of the stool sample is processed to yield a liquid sample. A processed stool sample is a liquid sample obtained upon extraction of a stool sample with an extraction buffer.

The processing of the stool sample is accomplished by an extraction buffer that is optimized for the task. It should fulfill at least three basic requirements: It should liberate the analyte of interest from the stool matrix. It should stabilize the free analyte. It should minimize the interference of the stool matrix in the subsequent detection of the analyte. Since marker combinations might hold additional diagnostic potential, an optimized buffer should not only be applicable for one specific biomarker but for all analytes of interest. The extraction buffer may contain urea to improve the homogenization and extraction of the stool sample. Ca²⁺ may be included for stabilization of a Ca²⁺-binding protein. Since S100 proteins are known to be Ca²⁺-binding proteins the extraction buffer preferably will contain Ca²⁺-ions to stabilize such proteins. Weak chelating agents should be used that on the one hand can break ion bridges between Ca²⁺ -binding proteins and the stool matrix. However, on the other hand, these Ca²⁺-complexing agents have to bind Ca²⁺-ions sufficiently weak to avoid stripping of Ca²⁺-ions from S100A12. Preferably nitrilotriacetic acid or citrate are used as chelators in a stool extraction buffer. An optimized and preferred extraction buffer contains urea, Ca²⁺-ions and a chelator. Preferably the chelator is selected from the group consisting of nitrilotriacetic acid or citrate. An optimized extraction buffer is e.g. used in the processing of a stool sample as shown in Example 4b.

It is most convenient to use an optimized extraction buffer in combination with a tailor-made stool sampling device. In a most convenient way, an individual collects a defined amount of stool sample and transfers it directly into the collection prefillcd with the stabilizing extraction buffer. This convenient mode of sampling and extraction enables the transport of the specimen to a diagnostic laboratory without degradation of the analyte. Since the extraction of the stool sample can be achieved directly in the sampling device the necessary handling and transfer procedures are reduced.

Several recent developments have focused on devices that facilitate the sampling and handling of a stool sample. EP 1 366 715 discloses a special collection tube for collection of a stool sample. This extraction tube essentially comprises (a) a container body that is hollow on the inside, open at the top, and able to receive a buffer solution, (b) a top cap provided with a threaded small rod for collection of fecal samples, said threaded small rod protruding axially inside the container body, when the top cap is applied to the top end of the container body, and (c) a dividing partition provided, in an intermediate position, inside said container body so as to separate a top chamber from a bottom chamber inside said container body, said dividing partition having an axial hole suitable to allow the passage of said threaded small rod, so as to retain the excess feces in said top chamber and allow the passage of the threaded part of the small rod into said bottom chamber. This extraction tube further has a container body that is open at the bottom and provided with a bottom cap which can be applied movably to the bottom end of the container body, so that said extraction tube can be used directly as a primary sampling tube to be inserted into a sample-holder plate of automatic analyzers, following removal of said bottom cap and overturning of said container body. The device disclosed in EP 1 366 715 allows for the convenient handling of a defined quantity of a stool sample and has the advantage that after appropriate extraction the tube may be directly placed into the sample-holder of an automatic analyzer.

A second example of a sophisticated stool sampling device that is appropriate for a convenient sampling and handling of a stool sample is described in WO 03/068398.

The stool sample is preferably used or processed directly after sampling or stored cooled or more conveniently stored frozen. Frozen stool samples can be processed by thawing, followed by dilution in an appropriate buffer, mixing and centrifugation. Supernatants are used as liquid sample for subsequent measurement of the marker S100A12.

An aliquot of the processed stool sample is incubated with the specific binding agent for S100A12 under conditions appropriate for formation of a binding agent S100A12-complex. Such conditions need not be specified, since the skilled artisan without any inventive effort can easily identify such appropriate incubation conditions.

As a final step according to the method disclosed in the present invention the amount of complex is measured and correlated to the diagnosis of CRC. As the skilled artisan will appreciate there are numerous methods to measure the amount of the specific binding agent S100A12-complex all described in detail in relevant textbooks (cf., e.g., Tijssen P., *supra,* or Diamandis et al. (eds.), Immunoassay, Academic Press, Boston (1996)).

Preferably S100A12 is detected in a sandwich type assay format. In such assay a first specific binding agent is used to capture S100A12 on the one side and a second specific binding agent, which is labeled to be directly or indirectly detectable is used on the other side.

Antibodies to S100A12 can also be used for assessment of CRC in other procedures, e.g., to detect colorectal cancer cells in situ, in biopsies, or in immunohistological staining procedures.

Preferably, an antibody to S100A12 is used in a qualitative (S100A12 present or absent) or quantitative (S100A12 amount is determined) immunoassay.

As mentioned above, it has surprisingly been found that S100A12 can be measured from a stool sample obtained from an individual sample. No tissue and no biopsy sample is required to apply the marker S100A12 in the diagnosis of CRC.

Whereas application of routine proteomics methods to tissue samples, e.g. by comparing healthy and cancerous tissue, leads to the identification of many potential marker candidates for the tissue/disease selected, these marker candidates only accidentally and in rare cases are found in the circulation. Surprisingly, the inventors of the present invention have been able to detect protein S100A12 in a stool sample. They have been able to demonstrate that the presence of S100A12 in such stool sample obtained from an individual can be correlated to the diagnosis of colorectal cancer.

It has also been found that the presence or absence of S100A12 in such stool sample obtained from an individual can be correlated to the presence or absence of colorectal cancer in a patient. The present invention also relates to a method for excluding colorectal cancer comprising the steps of a) providing a stool sample obtained from an individual, b) processing said sample to obtain a processed liquid sample, c) contacting said processed liquid sample with a specific binding agent for S100A12 under conditions appropriate for formation of a complex between said binding agent and S100A12, and d) using the absence of a complex in (c) as an indicator for the absence of colorectal cancer.

As the skilled artisan will appreciate, a positive result for S100A12 in a stool sample must not necessarily mean that the patient has CRC. However, a positive value for S100A12 in a stool sample should be considered a clear-cut indicator to warrant further and more sophisticated diagnostics. In a preferred embodiment a positive value for S100A12 as measured from a stool sample is used as an indicator that the patient should be offered further investigations, especially a virtual computed tomographic colonography (VCTC) or a colonoscopy. Preferably, a positive value in a stool sample is used as an indicator that colonoscopy as the next step in the (diagnostic) examination of a patient is warranted.

Measuring the level of protein S100A12 has proven very advantageous in the field of CRC. Therefore, in a further preferred embodiment, the present invention relates to use of protein S100A12 as a marker molecule in the diagnosis of colorectal cancer from a stool sample obtained from an individual.

The term marker molecule is used to indicate that the presence of or an increased level of the analyte S100A12 as measured from a processed stool sample obtained from an individual marks the presence of CRC. Obviously the marker S100A12 may be measured by any appropriate means an the presence or value measured used in the assessment of CRC.

As obvious to the skilled artisan, the present invention shall not be construed to be limited to the measurement of the full-length protein S100A12 of SEQ ID NO: 1. Physiological fragments of S100A12 can also be measured and used as a marker for CRC while practicing the present invention. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. As the skilled artisan will appreciate, S100A12 or fragments thereof may also be present as part of a complex. Such complex also may be used as a marker in the sense of the present invention. In addition, or in the alternative the S100A12 polypeptide may carry a post-translational modification, and such modified S100A12 may also serve as a marker of CRC.

Artificial fragments of S100A12 may be used, e.g. as a positive control in an immuno assay or as an immunogen. Artificial fragments preferably encompass a peptide produced synthetically or by recombinant techniques consisting of at least 6, 7, 8, 9, 10, 12, or at least 15 contiguous amino acids as derived from the sequence disclosed in SEQ ID NO:1. Preferably such artificial fragment comprises at least one epitope of diagnostic interest. Also preferred the artificial fragment comprises at least two epitopes of interest and is appropriate for use as a positive control in a sandwich immunoassay.

It is preferred to use the novel marker S100A12 in the early diagnosis of colorectal cancer. However, as the skilled artisan will appreciate S100A12, alike other markers, will also be of great advantage in the diagnosis and follow-up of patients already suffering from CRC at more advanced stages of tumor progression.

The S100A12 concentration closely correlates with tumor burden in CRC. The marker is therefore also suitable for the follow-up of CRC patients after treatment. In a preferred embodiment, the novel marker S100A12 is used in the follow-up of patients suffering from CRC. By measuring CRC regularly, e.g. at 3-monthly, 6-monthly or yearly intervals, tumor progression and/or as the case may be tumor recurrence can be assessed.

An increase or a re-appearance of S100A12 above the normal cut-off value are considered indicative for CRC tumor progression or for tumor recurrence, respectively. A further preferred embodiment therefore relates to a method of assessing by an in vitro measurement a patient suffering from colorectal cancer after surgery for removal of the cancerous lesion the method comprising the steps of a) providing a stool sample obtained from said patient, b) contacting said sample with a specific binding agent for S100A12 under conditions appropriate for formation of a complex between said binding agent and S100A12, c) determining the amount of complex formed in step (b) and d) correlating the amount of complex determined in (c) to a recurrence or progression of colorectal cancer. Measurement of S100A12 from a stool sample is especially helpful and in a preferred embodiment is used in the early detection of a CRC tumor recurrence within the gastrointestinal tract.

The colon as well as the rectum both are part of the gastrointestinal tract. Now that it has been shown that S100A12 most likely will be useful in the screening of patients for colorectal cancer, it is very likely that the presence of S100A12 in a stool sample may also be used as a diagnostic aid in the assessment of other types of gastrointestinal cancer. In a further preferred embodiment the present invention relates to the use of S100A12 as measured from a stool sample in the assessment of a gastrointestinal tumor. Preferably S100A12 as measured from a stool sample is also used in the assessment a stomach tumor. Measurement of S100A12 from a stool sample can be helpful and therefore represents a preferred embodiment according to the present invention in the early detection of a gastrointestinal tumor recurrence within the gastrointestinal tract.

The use of protein S100A12 itself, represents a significant progress to the challenging field of CRC diagnosis from stool. Combining measurements of S100A12 with other known markers, like hemoglobin or the hemoglobin-haptoglobin complex, or with other markers of CRC yet to be discovered, leads and may lead, respectively, to further improvements in the assessment of CRC. Therefore in a further preferred embodiment the present invention relates to the use of S100A12 as a marker molecule for colorectal cancer in combination with one or more other marker molecules for colorectal cancer in the diagnosis of colorectal cancer from a stool sample obtained from an individual. Preferred selected other CRC markers with which the measurement of S100A12 may be combined are TIMP-1, calprotectin, tumor M2 pyruvate kinase (M2-PK), hemoglobin and/or the hemoglobin-haptoglobin complex.

As a further preferred embodiment the present invention discloses the use of protein S100A12 as a marker molecule for colorectal cancer in combination with one or more other marker molecule(s) for colorectal cancer selected from the group consisting of hemoglobin/haptoglobin complex, hemoglobin, tissue inhibitor of metalloproteases 1 (TIMP-1), calprotectin, and tumor M2 pyruvate kinase (M2-PK).

In a preferred embodiment the present invention relates to the use of a marker combination comprising the markers S100A12 and TIMP-1 in the assessment of CRC, whereas both markers are measured from a stool sample.

In a preferred embodiment the present invention relates to the use of a marker combination comprising the markers S100A12 and hemoglobin in the assessment of CRC, whereas both markers are measured from a stool sample.

In a preferred embodiment the present invention relates to the use of a marker combination comprising the markers S100A12 and the hemoglobin/haptoglobin complex in the assessment of CRC, whereas both markers are measured from a stool sample.

In a preferred embodiment the present invention relates to the use of a marker combination comprising the markers S100A12 and tumor M2 pyruvate kinase (M2-PK) in the assessment of CRC, whereas both markers are measured from a stool sample.

In yet a further preferred embodiment the present invention relates to the use of a marker combination comprising the markers S100A12, TIMP-1 and hemoglobin in the assessment of CRC, whereas all three markers are measured from a stool sample.

In yet a further preferred embodiment the present invention relates to the use of a marker combination comprising the markers S100A12, TIMP-1 and the hemoglobin/haptoglobin complex in the assessment of CRC, whereas all three markers are measured from a stool sample.

TIMP-1 (=tissue plasminogen activator 1)

Matrix metalloproteinases (MMP's) play a pivotal role in cancer growth and spread, contributing to enzymatic degradation of the extracellular matrix. The naturally occurring inhibitors of MMP's, are called tissue inhibitors of MMP's or TIMP's. TIMP's form tight 1:1 stoichiometric complexes with the activated forms of the MMP's thereby inhibiting the catalytic activity of these enzymes.

A number of enzyme-linked immunoassays for the detection of TIMP-1 (Kodama, S., et al., Matrix 9 (1989) 1-6; Cooksley, S., et al., Matrix 10 (1990) 285-291; Clark, I.M., et al., Matrix 11 (1991) 76-85) and TIMP-2 (Fujimoto, N., et al., Clin. Chim. Acta 220 (1993) 31-45) have been described. These assays have been applied to body fluids, e.g. serum, plasma, amniotic fluid, cerebrospinal fluid and urine. No data can be found, in the art demonstrating the presence of TIMP-1 in a stool sample. No data are available in the art, indicative for the fact that the presence of TIMP-1 in a stool sample could be of clinical utility.

Diagnostic reagents in the field of specific binding assays, like immunoassays, usually are best provided in the form of a kit, which comprises the specific binding agent and the auxiliary reagents required to perform the assay. The present invention therefore also relates to an immunological kit comprising at least one specific binding agent for S100A12 and auxiliary reagents for measurement of S100A12.

Accuracy of a diagnostic test is often described by its receiver-operating characteristics (ROC) (see especially Zweig, M.H. and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) / (number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results) / (number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/l-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the curve of the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the AUC is to the perfect one (area = 1.0).

Clinical utility of the novel marker S100A12 has been assessed in comparison to and in combination with the established marker hemoglobin using a receiver operator characteristics analysis (ROC; Zweig, M.H. and Campbell, G., Clin. Chem. 39 (1993) 561-577). This analysis has been based on samples derived from well-defined patient cohorts as given in the examples section.

The diagnostic method based on measurement of S100A12 alone in comparison to the established marker hemoglobin alone has been found to have an at least as good a diagnostic accuracy (sensitivity/specificity profile) as demonstrated by the area under the curve.

The following examples, figures and sequence listing are provided to aid the understanding of the present invention, the true scope of which is set forth in the appended claims.

### Description of the Figure

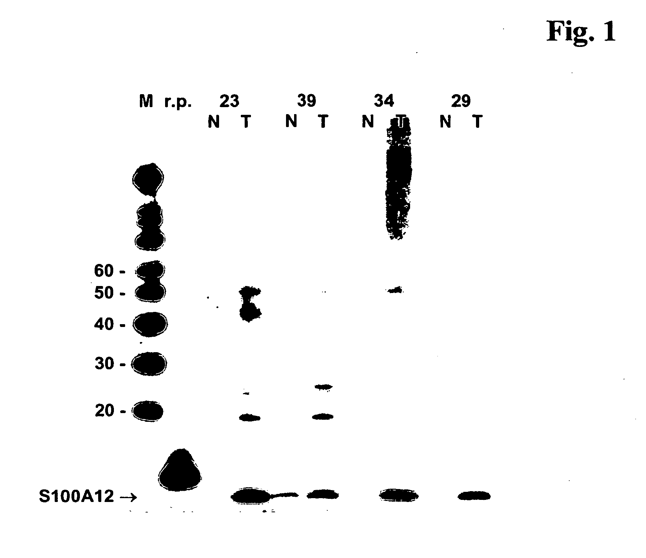

- **Figure 1:**: **Western Blot analysis**
Tumor and normal tissue of four CRC patients was analyzed by Western Blot using a polyclonal antibody against human S100A12. Recombinant protein expressed in E. coli as 6xHis-tagged protein was used as a positive control. The difference in the migration pattern of the recombinant protein is caused by the 6xHis-tag comprised therein. Normal tissue was recovered from the patients when the tumor was surgically removed. M: molecular weight marker; r.p.: recombinant protein; T: tumor tissue; N: normal tissue.
- **Figure 2:**: **ROC plot**
Plot of the receiver operator characteristics (ROC) for the assessment of 23 samples obtained from patients with CRC as compared to 18 samples obtained from healthy individuals is given.
- **Figure 3:**: **Scatter plots of S100A12 concentrations in CRC patients**
Effect of Ca²⁺-ions on the measurement of S100A12 in stool extracts. The stool extracts were diluted in the absence (figure 3a) or presence (figure 3b) of Ca²⁺ -ions. The detectable concentrations are higher in the presence of Ca²⁺-ions and are distributed over an increased dynamic range.

### Abbreviations

- ABTS: 2,2'-Azino-di-[3-ethylbenzthiazoline sulfonate (6)] diammonium salt
- BSA: bovine serum albumin
- cDNA: complementary DNA
- DMSO: dimethyl sulfoxide
- DTT: dithiothreitol
- EDTA: ethylene diamine tetraacetic acid
- ELISA: enzyme-linked immunosorbent assay
- ESI: electrospray ionization
- FCS: fetal calf serum
- IAA: iodoacetamid
- IgG: immunoglobulin G
- LC: liquid chromatography
- MS: mass spectroscopy
- MES: mesityl, 2,4,6-trimethylphenyl
- PAGE: polyacrylamide gel electrophoresis
- PBS: phosphate buffered saline
- PI: isoelectric point
- POD: horseradish peroxidase
- RTS: rapid translation system
- SDS: sodium dodecyl sulfate

### Example 1

### Identification of S100A12 as a potential colorectal cancer marker

### Sources of tissue

In order to identify tumor-specific proteins as potential diagnostic markers for colorectal cancer, analysis of three different kinds of tissue using proteomics methods is performed.

In total, tissue specimen from 10 patients suffering from colorectal cancer are analyzed. From each patient three different tissue types are collected from therapeutic resections: tumor tissue (>80% tumor) (T), adjacent healthy tissue (N) and stripped mucosa from adjacent healthy mucosa (M). The latter two tissue types serve as matched healthy control samples. Tissues are immediately snap frozen after resection and stored at -80°C before processing. Tumors are diagnosed by histopathological criteria.

### Tissue preparation

0.8-1.2 g of frozen tissue are put into a mortar and completely frozen by liquid nitrogen. The tissue is pulverized in the mortar, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton® glass homogenizer (20 x loose fitting, 20 x tight fitting). 3 ml of the homogenate are subjected to a sucrose-density centrifugation (10-60% sucrose) for 1 h at 4,500 x g. After this centrifugation step three fractions are obtained. The fraction on top of the gradient contains the soluble proteins and is used for further analysis.

### Sample preparation for LC-ESI-MSMS-analysis

The protein concentration of the soluble protein fraction is determined using Bio-Rad® protein assay (Cat.No. 500-0006; Bio-Rad Laboratories GmbH, München, Germany) following the instructions of the supplier's manual. To a volume corresponding to 200 µg of protein 4 ml reduction buffer (9 M urea, 2 mM DTT, 100 mM KH₂PO₄, pH 8.2 NaOH) is added and incubated for 1 h. The solution is concentrated to 250 µl in an Amicon^{®} Ultra 10 kD device (Millipore GmbH, Schwalbach, Germany). For alkylation the 250 µl are transferred into 1 ml alkylation buffer (9 M urea, 4 mM iodoacetamide, 100 mM KH₂PO₄, pH 8.2 NaOH), incubated for 6 h and subsequently concentrated in an Amicon^{®} Ultra 10 kD device to 250 µl. For washing 1 ml 9 M urea is added and again concentrated in an Amicon^{®} Ultra 10 kD device to 250 µl. Washing is repeated three-times.

For protease digestion the concentrated solution is diluted to 2.5 M urea and incubated with 4 µg trypsin (Proteomics grade, Roche Diagnostics GmbH, Mannheim, Germany) over night. The digestion is stopped by adding 1 ml 1% formic acid and analyzed.

### LC-ESI-MSMS-analysis

The tryptic digest (500 µl) is separated on a two-dimensional Nano-HPLC-System (Ultimate, Famos, Switchos; LC Packings, Idstein, Germany) consisting of a SCX and a RP Pepmep C18 column (LC Packings, Idstein, Germany). The 11 SCX fractions (step elution with 0, 5, 10, 25, 50, 100, 200, 300, 400, 500, 1.500 mM NH₄Ac) where successively further separated on the RP column with a 90 min gradient (5-95% acetonitrile) and online analyzed using data dependent scans with an ESI-MS ion trap (LCQ deca XP; Thermo Electron, Massachusetts, USA; see Table 2 for parameters). For each sample three runs are performed. The raw data are processed with Bioworks 3.1 software (Thermo Electron, Massachusetts, USA) using the parameters listed in Table 2. The resulting lists of identified peptides and proteins from replicate runs where combined.

The protein S100A12 is identified by aid of the partial sequences given in Table 3.

### Detection of S100A12 as a potential marker for colorectal cancer

For each patient the identified proteins and the number of corresponding peptides from the tumor sample are compared to the accordant results from adjacent normal tissue and from stripped normal mucosa tissue. By this means, protein S100A12 is found to be specifically expressed or strongly overexpressed in tumor tissue and not or less detectable or less strongly expressed in healthy control tissue. It therefore - amongst many other proteins - qualifies as a candidate marker for use in the diagnosis of colorectal cancer.

The protein S100A12 was strongly over-represented in tumor tissue from patients suffering from colorectal cancer. The following peptide sequences of the protein S100A12 were identified with Bioworks 3.1 form LCQ-MS²-data in tumor tissue:

**Table 2: MS/MS-data acquisition and Bioworks 3.1 search parameters**

| **MSMS-data acquisition** | **MS exclusion** | **350-2,000 Da for precursor ions** |
|---|---|---|
| | Repeat count | 2 |
| | Repeat duration | 0.25 min |
| | Exclusion list size | 25 |
| | Exclusion duration | 5 min |
| | Exclusion mass width | low 0.5 Da, high 1.5 Da |
| Bioworks | Number of ions | 35 |
| | Minimal ion intensity | 100,000 counts |
| | Precursor mass tolerance | 1.2 Da |
| | Fragment mass tolerance | 1.4 Da |
| | X_{corr} | > 2; 2.5; 3 (z = 1; 2; 3) |
| | dCn | > 0.1 |
| | Sp | > 500 |
| Databases | | Swissprot; Humangp (assembled by Roche Bioinformatics) |

**Table 3: Sequences found**

| | |
|---|---|
| i | TKLEEHLEGIVNIFHQYSVR |
| ii | GHFDTLSKGELK |

The above two peptide sequences have been identified as partial sequences of S100A12. Sequence (i) represents the amino acid positions from amino acid 2 to amino acid 21 of SEQ ID NO:1 and sequence (ii) represents the amino acid positions from amino acid 23 to amino acid 34 of SEQ ID NO:1, respectively.

### Example 2

### Generation of antibodies to the colorectal cancer marker protein S100A12

Polyclonal antibody to the colorectal cancer marker protein S100A12 is generated for further use in the measurement of S100A12 in serum, plasma, blood and especially in a stool sample. Such measurements of S100A12 e.g. are performed by immunodetection assays, e.g. Western Blotting and ELISA.

### Recombinant protein expression in E. coli

In order to generate antibodies to S100A12, recombinant expression of the protein is performed for obtaining immunogens. The expression is done applying a combination of the RTS 100 expression system and E.coli. In a first step, the DNA sequence is analyzed and recommendations for high yield cDNA silent mutational variants and respective PCR-primer sequences are obtained using the "ProteoExpert RTS E.coli HY" system. This is a commercial web based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA and for in-vitro transcription and expression of the nucleotide sequence coding for the S100A12 protein is used. For Western-blot detection and later purification, the expressed protein contains a His-tag. The best expressing variant is identified. All steps from PCR to expression and detection are carried out according to the instructions of the manufacturer. The respective PCR product, containing all necessary T7 regulatory regions (promoter, ribosomal binding site and T7 terminator) is cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et al., Methods Enzymol. 185 (1990) 60-89) and the transformed bacteria are cultivated in a 11 batch for protein expression.

Purification of His-S100A12 fusion protein is done following standard procedures on a Ni-chelate column. Briefly, 1l of bacteria culture containing the expression vector for the His-S100A12 fusion protein is pelleted by centrifugation. The cell pellet is resuspended in lysis buffer, containing 50 mM Na-phosphate, pH 8.0, 300 mM NaCl, 1 mg/mL Lysozyme, 2x Complete (EDTA-free) and DNase, followed by cell disruption using a French press (Model: IUL Basic Z). Insoluble material is pelleted by high speed centrifugation and the supernatant is applied to a Ni-chelate chromatographic column. The column is washed with several bed volumes of washing buffer (50 mM Na-phosphate, pH 8.0, 300 mM NaCl, 20 mM imidazole. Finally, bound antigen is isolated using an elution buffer containing 50 mM Na-phosphate, pH 8.0, 300 mM NaCl with a linear gradient of 20 to 500 mM imidazole.

### Production of monoclonal antibodies against the S100A12

### a) Immunization of mice

12 week old A/J mice are initially immunized intraperitoneally with 100 µg S100A12. This is followed after 6 weeks by two further intraperitoneal immunizations at monthly intervals. In this process each mouse is administered 100 µg S100A12 adsorbed to aluminum hydroxide and 10⁹ germs of *Bordetella pertussis.* Subsequently the last two immunizations are carried out intravenously on the 3rd and 2nd day before fusion using 100 µg S100A12 in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1*10⁸ spleen cells of the immunized mouse are mixed with 2x10⁷ myeloma cells (P3X63-Ag8-653, ATCC CRL1580) and centrifuged (10 min at 300 x g and 4°C.). The cells are then washed once with RPMI 1640 medium without fetal calf serum (FCS) and centrifuged again at 400 x g in a 50 ml conical tube. The supernatant is discarded, the cell sediment is gently loosened by tapping, 1 ml PEG (molecular weight 4,000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 x g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor.

After ca. 10 days the primary cultures are tested for specific antibody. S100A12-positive primary cultures are cloned in 96-well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1x10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-596).

### Generation of polyclonal antibodies

### a) Immunization

For immunization, a fresh emulsion of the protein solution (100 µg/ml protein S100A12) and complete Freund's adjuvant at the ratio of 1:1 is prepared. Each rabbit is immunized with 1 ml of the emulsion at days 1, 7, 14 and 30, 60 and 90. Blood is drawn and resulting anti-S100A12 serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8,000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13,000 x g, 15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin-N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies are biotinylated according to the same procedure.

### Digoxygenylation of polyclonal rabbit IgG

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex® 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 3

### Western Blot detection of S100A12 in cancer and healthy tissue, respectively

Tissue lysates from tumor samples and healthy control samples are prepared as described in Example 1, "Tissue preparation".

SDS-PAGE and Western-Blotting are carried out using reagents and equipment of Invitrogen, Karlsruhe, Germany. For each tissue sample tested, 10 µg of tissue lysate are diluted in reducing NuPAGE^{®} (Invitrogen) SDS sample buffer and heated for 10 min at 95°C. Samples are run on 4-12% NuPAGE^{®} gels (Tris-Glycine) in the MES running buffer system. The gel-separated polypeptides are blotted onto nitrocellulose membranes using the Invitrogen XCell II™ Blot Module (Invitrogen) and the NuPAGE^{®} transfer buffer system. The membranes are washed 3 times in PBS/0.05% Tween-20 and blocked with Roti^{®}-Block blocking buffer (A151.1; Carl Roth GmbH, Karlsruhe, Germany) for 2 h. The primary antibody, polyclonal rabbit anti-S100A12 serum (generation described in Example 2), is diluted 1:10,000 in Roti^{®}-Block blocking buffer and incubated with the membrane for 1 h. The membranes are washed 6 times in PBS/0.05% Tween-20. The specifically bound primary rabbit antibody is labeled with an POD-conjugated polyclonal sheep anti-rabbit IgG antibody, diluted to 10 mU/ml in 0.5 x Roti^{®}-Block blocking buffer. After incubation for 1 h, the membranes are washed 6 times in PBS/0.05% Tween-20. For detection of the bound POD-conjugated anti-rabbit antibody, the membrane is incubated with the Lumi-Light^{PLUS} Western Blotting Substrate (Order-No. 2015196, Roche Diagnostics GmbH, Mannheim, Germany) and exposed to an autoradiographic film.

When tumor and normal tissue derived from four individuals diagnosed with CRC is blotted as described, high expression of S100A12 protein is detected in tumor tissue while no or much less S100A12, respectively, is found in normal tissue (Figure 1).

### Example 4

### Processing of stool specimen

### a) Detergent extraction

About 1 g of stool per sample is collected by the patients in a special sampling device from Sarstedt, Germany, Order-No. 80.623.022, frozen and stored at -70°C. For analysis, the stool samples are thawed, approximately 100 mg of stool sample are diluted tenfold with a phosphate buffer, pH 7.4, supplemented with Mini Complete EDTA-free, a protease inhibitor cocktail from Roche, Germany (order-no.: 11 873 580):

| | |
|---|---|
| Na₂HPO₄ | 53.4 mM |
| KH₂PO₄ | 12.3 mM |
| NaN₃ | 0.1 % |
| Na₂EDTA | 1.07 mM |
| Chicken albumen | 1.0 % |
| Nonidet P-40 | 0.5 % |

The samples are incubated for 1 h at room temperature with continuous shaking and for one more hour without shaking at 4 °C. After centrifugation for 15 min at 3.000 x g, the supernatant is pipetted off and filtered using a membrane filter with a pore size of 5 µm. The samples are aliquoted and stored at -70 °C. An aliquot of this processed stool sample is used for quantitation of S100A12 by ELISA.

### b) Urea extraction

To improve the measurement of S100A12 and other markers of interest in stool samples an "optimized extraction buffer" is used. For the processing of the stool samples the extraction buffer is freshly prepared by adding a protease inhibitor cocktail (Mini Complete EDTA-free, Roche, Germany) to the following buffer:

| | |
|---|---|
| TRIS | 0.10 mol/l, pH 8.0 |
| Citric acid | 0.10 mol/l |
| Urea | 1.0 mol/l |
| CaCl₂ | 0.01 mol/l |
| BSA | 0.50 % |

The stool samples are thawed and 50 - 100 mg of each sample are transferred to a fecal sample preparation kit (cat.-no.: 10745804 Roche, Germany). Optimized extraction buffer is added according to the weight of the stool samples to give a 50-fold dilution. The samples are vigorously mixed on an orbital shaker for 30 minutes, transferred to a 10 ml tube (Sarstedt, Germany) and centrifuged at 1200 g for 10 minutes. The supernatant is filtered using a 5 µm cut-off filter (Ultrafree^{®}-CL, Millipore, Germany), aliquoted and stored for further analysis at -70 °C. These stool extracts are suitable for all biomarkers of interest in this study.

### Example 5

### ELISA for the measurement of S100A12 in human stool specimen

A sandwich ELISA is developed for the determination of S100A12 from stool. Polyclonal rabbit antibodies are produced by immunization with recombinant full length S100A12 expressed in E. coli. The purified IgG fraction of the antisera is biotinylated or digoxigenylated to used to establish a sandwich ELISA using 96-well streptavidin plates (Streptawell, HighBind, Roche, Germany). Stool extracts are diluted 1:25 in sample dilution buffer (100 mM Tris, pH 8.0, 2 mM CaCl₂, 1.5 % KCL, 0.3 % Triton X-100, 0.2 % Casein, 2 % sucrose) and 50 µl of sample or standard are transferred to each well. Subsequently the formation of the antigen-antibody complex is started by adding 50 µl of an antibody mixture containing 0.5 µg/ml biotinylated polyclonal antibody PAB<S100A12>-Bi and 0.5 µg/ml digoxigenylated polyclonal antibody PAB<S100A12>-Dig in assay buffer (PBS, pH 7.4, 0.1 % bovine IgG, 0.9 % NaCl, 0.5 % Thesit, 1 % PEG 40.000, 0.1% bovine IgG, 0.025% bovine gamma globulin acetylated). The plates are incubated for 60 minutes, washed three times with 350 µl washing buffer per well (100 mM PBS, pH 7.4, 0.05% TWEEN-20). Thereafter 100 µl of 25 mU/ml MAB<Dig>-POD conjugate (Roche Diagnostics, Germany) per well are added and incubated for another 60 minutes. After three times washing with 350 µl washing buffer per well, 100 µl ABTS solution (Roche Diagnostics, Germany) per well are added and the plates incubated for 60 minutes. The absorbance is measured at 405/620 nm using an ELISA reader (SLT.Spectra II). Recombinant full-length S100A12 is used for calibration.

### Example 6

### Analyte stability in urea extract

S100A12 appears to be more stable than hemoglobin in stool extracts prepared using the extraction method described in 4b. When stool extracts are stored for 1 or 3 days at room temperature the average recovery for S100A12 is higher and appears to show less scatter than the average recovery of hemoglobin. Of the 20 samples used to assess the stability two are hemoglobin negative:

**Table 4: Recovery after temperature stress**

| | **N** | **Concentration range of samples** | **Recovery after 1 d RT** | **Recovery after 3 d RT** |
|---|---|---|---|---|
| S100A12 | 20 | 21-67,663 ng/g | 87 % (± 15 %) | 73 % (± 20 %) |
| Hemoglobin | 18 | 0.32 - 10,364 µg/g | 79 % (± 23 %) | 59 % (± 30 %) |

### Example 7

### Clinical utility of S100A12 in colorectal cancer

The clinical utility of S100A12 is assessed by analyzing stool samples obtained from well-characterized patient cohorts. For each patient two stool samples from the same bowel movement are measured and the concentrations are analyzed. The correlation of both concentrations is assessed by Pearson's correlation coefficient revealing a close correlation. To improve the sensitivity of the assay the maximum concentration measured in one of the two paired samples is used for further analysis. The diagnostic value of S100A12 is evaluated by ROC analysis according to Zweig et al (supra).

The stool samples of the first two study populations are extracted according to Example 4a. For dilution phosphate buffered saline pH 7.4, 1.0 mM CaCl₂, 1.2 mM nitrilotriacetic acid, 0.1 % bovine serum albumin is used. The stool samples of the third study population are extracted according to Example 4b and diluted with the sample dilution buffer as given in Example 5. Despite different extraction procedures, the ELISA procedure used for the measurement of S100A12 is identical for all patient populations (cf. Example 5).

### First study population:

Stool samples from 18 CRC-free patients, 10 patients diagnosed as adenoma positive by colonoscopy, and 23 patients diagnosed with progressed CRC classified as UICC stages III and IV are obtained. S100A12 is measured as described above in a stool sample obtained from each of these individuals.

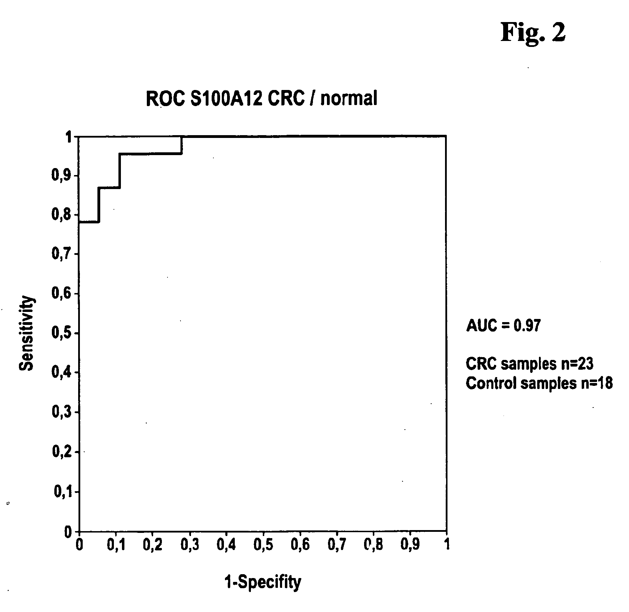

ROC-analysis is performed according to Zweig, M. H., and Campbell, *supra.* Discriminatory power for differentiating patients in the CRC group from healthy individuals as measured by the area under the curve is found to be 97 % for CRC vs. healthy controls (Figure 2), 85 % for CRC + adenoma vs. healthy controls and 57 % for adenoma vs. healthy controls, respectively.

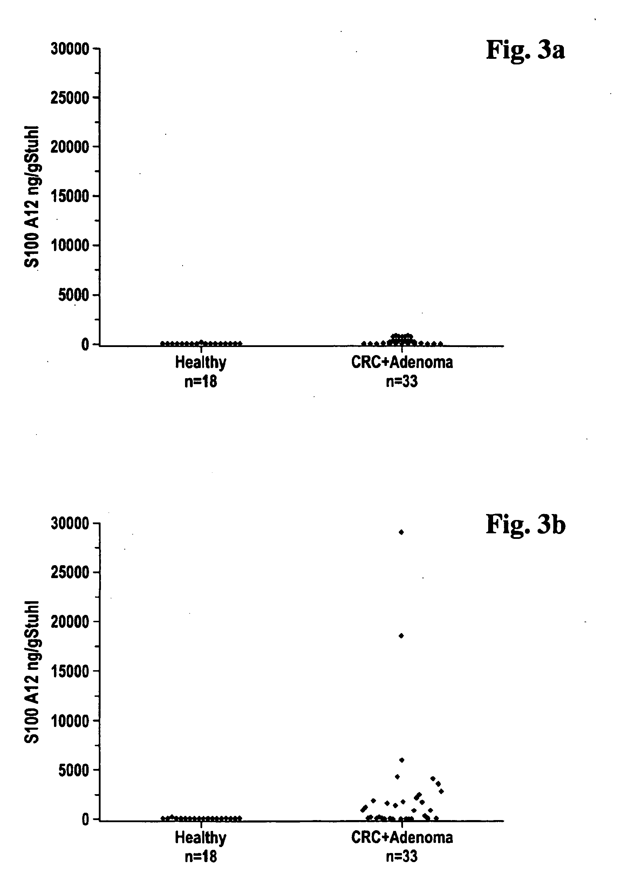

The positive effect of the presence of Ca²⁺-ions on the determination of S100A12 from stool is illustrated in Figure 3. When the stool extracts are diluted in phosphate buffered saline pH 7.4, 0.1 % bovine serum albumin in the absence of both CaCl₂ and nitrilotriacetic acid lower concentrations of S100A12 are detectable (Figure 3a) as compared to the S100A12 concentrations measured in the presence of CaCl₂ and nitrilotriacetic acid (Figure 3b). The smaller dynamic range of the ELISA in the absence of Ca²⁺ leads also to a reduced AUC of the ROC plot of 94 % for CRC vs. healthy controls while with the improved buffer system an AUC of 97 % is observed. If CRC + adenoma vs. healthy controls is considered the AUC is 81 % without Ca²⁺ and 85 % in the presence of CaCl₂ and nitrilotriacetic acid, respectively.

### Second, enlarged, study population:

Stool samples are obtained from 10 patients diagnosed as adenoma positive by colonoscopy, 50 patients diagnosed as CRC (9 classified as UICC stage I; 4 classified as UICC stage II; 21 classified as UICC stage III; 13 classified as UICC stage IV, 2 classified as UICC stage I to III, i.e. not IV; and 1 CRC without staging) and 50 controls (7 from patients with other GI-disease, i.e. not CRC; 16 from patients with diverticulosis; 8 from donors classified as GI-healthy; and 19 from patients suffering from hemorrhoids). S100A12 is measured as described above in a stool sample obtained from each of these individuals.

ROC-analysis is performed according to Zweig, M. H., and Campbell, *supra.* Discriminatory power for differentiating patients in the CRC group from healthy individuals as measured by the area under the curve is found to be 90 % for CRC vs. controls, 86 % for CRC + adenoma vs. healthy controls and 66 % for adenoma vs. healthy controls, respectively. This indicates that even the early UICC stages are detected if S100A12 is measured from a stool sample.

### Third study population

S100A12 is measured in a more extensive, third study population (for patient characteristics cf.: Table 5). A high number of clinically well-characterized stool samples is prospectively collected in the frame-work of multi-center study. The patients (undergoing a colonoscopy) for the control collective are recruited at gastroenterology units and representing an average-risk screening population. Patients with inflammatory bowel diseases and with any kind of adenoma are excluded from the control collective. Due to the low prevalence of colorectal cancer patients in a screening population, the samples from cancer patients are collected at different surgery units. The diagnosis of colorectal cancer is confirmed by a physician also providing the pathological staging for each cancer patient. To assess any bias that might be introduced by the common diagnostic work-up of patients by a guaiac-based FOBT prescreening a sub-collective without prior guaiac-based FOBT is also collected. All patients that are detected due to visible rectal bleeding are excluded from this sub-collective as well, because these would introduce a bias to the advantage of hemoglobin.

Stool samples are obtained from 252 control individuals. Of these 135 are confirmed by colonoscopy to be GI-healthy, while the remaining control samples cover several relevant GI-diseases. The CRC population includes 186 CRC samples from UICC stages I - IV (Table 6). For 38 CRC patients the exact staging is not known. Therefore the CRC patients with a defined UICC-stage in Table 6 do not sum-up to a total of 186 patients. In total 101 CRC patients without pre-screening by FOBT are evaluated.

S100A12 is measured as described above in a stool sample obtained from each of these individuals. ROC-analysis is performed according to Zweig, W. H., and Campbell, *supra.* Discriminatory power for differentiating patients in the CRC group from control individuals as measured by the area under the curve (Table 6) is found to be 94 % for CRC vs. all controls and as well 94 % for CRC without FOBT pre-screening vs. controls. Grouping the CRC patients by disease stage reveals an area under the curve of 90 % for UICC-stage I and 96 - 97 % for UICC-stages II - IV. No evidence for a bias by FOBT pre-screening is detected since the area under the curve of these patients is identical to the area under the curve of the total CRC population.

**Table 5: Patient characteristics of the third study population**

| | **Total number** | **Age (year)** | **Gender (female/male)** |
|---|---|---|---|
| Controls | 252 | 63,0 +/- 8,0 | 151/101 |
| - Healthy Controls (no evidence of any bowel disease) | 132 | 62,3 +/- 6,8 | 81/51 |
| - Hemorrhoids | 28 | 60,1 +/- 7,1 | 13/15 |
| - Diverticulosis | 73 | 64,7 +/- 9,5 | 46/27 |
| - Hyplastic polyps | 14 | 67,9 +/- 9,9 | 8/6 |
| - Other GI diseases | 5 | 59,2 +/- 6,7 | 3/2 |
| CRC (all samples) | 186 | 66.1 +/- 11.9 | 93/93 |
| CRC (subcollective w/o blood/FOBT) | 101 | 68,4 +/- 11,5 | 48/53 |

**Table 6: ROC analysis of a third study population**

| **Sample panel** | **N** | **AUC %** |
|---|---|---|
| CRC samples all | 186 | 94 |
| CRC samples w/o blood/FOBT | 101 | 94 |
| UICC stage I | 39 | 90 |
| UICC stage II | 38 | 97 |
| UICC stage III | 42 | 96 |
| UICC stage IV | 29 | 97 |

### Example 8

### Combinations of S100A12 with other stool markers

Combinations of S100A12 with other biomarkers from stool extracts are evaluated. The markers hemoglobin, the hetero-complex of hemoglobin with haptoglobin, Calprotectin, Time-1, M2-PK and CEA are measured using commercial ELISAs. Assays for measurement of hemoglobin, hemoglobin/ haptoglobin and calprotectin are obtained from R-Biopharm, Germany. The Calpro Calprotectin ELISA is manufactured by Calpro SA, Norway, and is marketed outside of Germany as PhiCal™ Test. The assay for measurement of CEA is obtained from Roche Diagnostics, Germany. The assay for M2-PK is supplied by Schebo Biotech, Germany, and the assay for TIMP-1 by R&D Systems, USA, respectively. While some of the assays are intended for measurements in stool extracts, for two assays, namely CEA and TIMP-1, stool is not a commonly used sample material. Hence, the assays have to be adjusted to the measurement of the corresponding analyte in a sample representing an extracted stool specimen. The samples (stool extracts) are prediluted 20-fold for CEA determinations, but otherwise the assay is run according to the manufacturers recommendations. Similarly the samples are prediluted 3-fold for the measurement of TIMP-1 without changing the assay procedure itself.

To test if a marker combination will improve the diagnosis of CRC, the markers are combined by Bayes Logistic Regression (BLR). In the BLR algorithm for the evaluation of marker combinations a Gaussian prior is used and implemented in the BBR-Software of Alexander Genkin, David D. Lewis, and David Madigan (Large-scale Bayesian logistic regression for text categorization. Technometrics,). The following settings are used: no automatic feature selection, prior variance fixed at 0.05, no threshold-tuning, and input standardization by normalization. For the numerical process the default settings with a convergence threshold of 0.0005, 1000 iterations and no-accuracy-mode are retained unchanged. The results with the basic algorithm get evaluated by 100 runs in a Monte-Carlo cross-validation design. In each run, two-third of all cases and controls, respectively, are selected as training set via the Matlab^{®} R2006a in-built function randsample with starting value 19022007 for the default random number generator. The basic algorithm is applied on the training set to generate a diagnostic rule. A threshold on the estimated posterior case-probabilities is determined on the controls of the training set to achieve a specificity of 95% or 98%, respectively. The diagnostic rule is then applied to the other third of the data to estimate sensitivity and specificity at the given threshold.

To avoid any bias for hemoglobin or hemoglobin / haptoglobin only the 101 CRC patients without prior FOBT prescreening are used in the assessment. For a screening assay not only the AUC of the ROC plot is relevant. A quite critical requirement in a screening setting is a good enough sensitivity at a high specificity. High specificity is crucial because a low specificity will cause a high number of false positive results accompanied by unnecessary follow-up procedures and distress for the patients. Table 7 summarizes the AUC values of the evaluation as well as the sensitivities at a preset specificity of 95% and 98%, respectively. When some of the individual markers measured are combined by BLR the AUC values for the diagnosis of CRC are very similar within the range of +/- 1%. On the other hand overall sensitivity in detection of CRC can be significantly improved by combination of S100A12 with other markers, particularly at a specificity level of 98%. While S100A12 alone has a sensitivity of 67%, the marker combination including the two markers S100A12 and the hemoglobin-haptoglobin complex exhibits a sensitivity of 79% and the best marker combination, including three markers, S100A12, hemoglobin-haptoglobin complex and TIMP-1, shows a further increase to 82% in sensitivity at the same high level of specificity. These marker combinations are considered very important in order to detect CRC, especially CRC at early stages. As obvious from Table 7, particularly a colorectal cancer at stage I is detected at a much higher rate by use of a marker combination. Use of the marker S100A12 is key to the improved ROC obtained with these marker combinations.

**Table 7: Marker combinations for the detection of CRC**

| Marker combination | N | S100A12 | S100A12 and Hb-Hp | S100A12 and Hb | S100A12 and TIMP-1 | S100A12 and calprotectin | S100A12 and M2-PK | S100A12 and CEA |
|---|---|---|---|---|---|---|---|---|
| AUC % | 101 | 95 | 96 | 96 | 94 | 94 | 95 | 94 |
| Sensitivity at 95 % Spec. | 101 | 82 | 88 | 88 | 79 | 76 | 82 | 82 |
| Sensitivity at 98 % Spec. | 101 | 67 | 79 | 76 | 73 | 67 | 73 | 67 |
| UICCI Sensitivity at 98 % Spec | 22 | 32 | 55 | 55 | 45 | | | |
| UICC II Sensitivity at 98 % Spec | 27 | 81 | 81 | 85 | 85 | | | |
| UICC III Sensitivity at 98 % Spec | 28 | 71 | 79 | 79 | 79 | | | |
| UICC IV Sensitivity at 98 % Spec | 24 | 71 | 87 | 87 | 75 | | | |

## Claims

1. A method for the diagnosis of colorectal cancer comprising the steps of
a) providing a stool sample obtained from an individual,
b) contacting said sample with a specific binding agent for S100A12 under conditions appropriate for formation of a complex between said binding agent and S100A12,
c) determining the amount of complex formed in step (b) and
d) correlating the amount of complex determined in (c) to the diagnosis of colorectal cancer.

2. A method for the diagnosis of colorectal cancer comprising the steps of
a) providing a stool sample obtained from an individual,
b) contacting said sample with a specific binding agent for S100A12 under conditions appropriate for formation of a complex between said binding agent and S100A12,
c) contacting said sample with a specific binding agent for a second marker selected from the group consisting of hemoglobin/haptoglobin complex, hemoglobin, tissue inhibitor of metalloproteases 1 (TIMP-1), and tumor M2 pyruvate kinase (M2-PK) under conditions appropriate for formation of a complex between said binding agent and the second marker,
d) detection the amount of complex formed in steps (b) and (c) and
e) correlating the amount of complex determined in step (d) to the diagnosis of colorectal cancer.

3. The method according to claim 2, wherein said second marker is hemoglobin.

4. The method according to claim 2, wherein said second marker is the hemoglobin / haptoglobin complex.

5. The method according to claim 2, wherein said second marker is TIMP-1.

6. The method according to claim 2, wherein said second marker is M2-PK.

7. Use of protein S100A12 as a marker molecule in the diagnosis of colorectal cancer from a stool sample obtained from an individual.

8. Use of protein S100A12 as a marker molecule in the early diagnosis of colorectal cancer from a stool sample obtained from an individual.

9. Use of protein S100A12 as a marker molecule for colorectal cancer in combination with one or more other marker molecule(s) for colorectal cancer selected from the group consisting of hemoglobin/haptoglobin complex, hemoglobin, tissue inhibitor of metalloproteases 1 (TIMP-1), and tumor M2 pyruvate kinase (M2-PK) in the diagnosis of colorectal cancer from a stool sample obtained from an individual.

## Patentansprüche

1. Verfahren zur Diagnose von Kolorektalkarzinom mit den folgenden Schritten:
a) Bereitstellen einer von einem Individuum erhaltenen Stuhlprobe;
b) Inkontaktbringen der Probe mit einem spezifischen Bindungsmittel für S100A12 unter für die Ausbildung eines Komplexes zwischen dem Bindungsmittel und S100A12 geeigneten Bedingungen;
c) Bestimmen der Menge an in Schritt (b) gebildetem Komplex und
d) Korrelieren der in Schritt (c) bestimmten Menge an Komplex mit der Diagnose von Kolorektalkarzinom.

2. Verfahren zur Diagnose von Kolorektalkarzinom mit den folgenden Schritten:
a) Bereitstellen einer von einem Individuum erhaltenen Stuhlprobe;
b) Inkontaktbringen der Probe mit einem spezifischen Bindungsmittel für S100A12 unter für die Ausbildung eines Komplexes zwischen dem Bindungsmittel und S100A12 geeigneten Bedingungen;
c) Inkontaktbringen der Probe mit einem spezifischen Bindungsmittel für einen zweiten Marker, ausgewählt aus der Gruppe bestehend aus Hämoglobin/Haptoglobin-Komplex, Hämoglobin, TIMP-1 (Tissue Inhibitor of Metalloproteases 1) und Tumor-M2-Pyruvatkinase (M2-PK), unter für die Ausbildung eines Komplexes zwischen dem Bindungsmittel und dem zweiten Marker geeigneten Bedingungen;
d) Nachweis der Menge an in den Schritten (b) und (c) gebildetem Komplex und
e) Korrelieren der in Schritt (d) bestimmten Menge an Komplex mit der Diagnose von Kolorektalkarzinom.

3. Verfahren nach Anspruch 2, wobei es sich bei dem zweiten Marker um Hämoglobin handelt.

4. Verfahren nach Anspruch 2, wobei es sich bei dem zweiten Marker um den Hämoglobin/Haptoglobin-Komplex handelt.

5. Verfahren nach Anspruch 2, wobei es sich bei dem zweiten Marker um TIMP-1 handelt.

6. Verfahren nach Anspruch 2, wobei es sich bei dem zweiten Marker um M2-PK handelt.

7. Verwendung des Proteins S100A12 als Markermolekül bei der Diagnose von Kolorektalkarzinom aus einer von einem Individuum erhaltenen Stuhlprobe.

8. Verwendung des Proteins S100A12 als Markermolekül bei der Frühdiagnose von Kolorektalkarzinom aus einer von einem Individuum erhaltenen Stuhlprobe.

9. Verwendung des Proteins S100A12 als Markermolekül für Kolorektalkarzinom in Kombination mit einem weiteren Markermolekül bzw. mehreren weiteren Markermolekülen für Kolorektalkarzinom, ausgewählt aus der Gruppe bestehend aus Hämoglobin/Haptoglobin-Komplex, Hämoglobin, TIMP-1 (Tissue Inhibitor of Metalloproteases 1) und Tumor-M2-Pyruvatkinase (M2-PK), bei der Diagnose von Kolorektalkarzinom aus einer von einem Individuum erhaltenen Stuhlprobe.

## Revendications

1. Procédé pour le diagnostic du cancer colorectal comprenant les étapes de
a) fourniture d'un échantillon de selles obtenu d'un individu,
b) mise en contact dudit échantillon avec un agent de liaison spécifique de S100A12 dans des conditions appropriées à la formation d'un complexe entre ledit agent de liaison et S100A12,
c) détermination de la quantité de complexe formé à l'étape (b) et
d) corrélation de la quantité de complexe déterminée en (c) au diagnostic de cancer colorectal.

2. Procédé pour le diagnostic du cancer colorectal comprenant les étapes de
a) fourniture d'un échantillon de selles obtenu d'un individu,
b) mise en contact dudit échantillon avec un agent de liaison spécifique de S100A12 dans des conditions appropriées à la formation d'un complexe entre ledit agent de liaison et S100A12,
c) mise en contact dudit échantillon avec un agent de liaison spécifique d'un deuxième marqueur choisi dans le groupe constitué par le complexe hémoglobine/haptoglobine, l'hémoglobine, l'inhibiteur tissulaire de métalloprotéases 1 (TIMP-1), et la M2 pyruvate kinase tumorale (M2-PK) dans des conditions appropriées à la formation d'un complexe entre ledit agent de liaison et le deuxième marqueur,
d) détection de la quantité de complexe formé aux étapes (b) et (c) et
e) corrélation de la quantité de complexe déterminée à l'étape (d) au diagnostic de cancer colorectal.

3. Procédé selon la revendication 2, dans lequel ledit deuxième marqueur est l'hémoglobine.

4. Procédé selon la revendication 2, dans lequel ledit deuxième marqueur est le complexe hémoglobine/haptoglobine.

5. Procédé selon la revendication 2, dans lequel ledit deuxième marqueur est le TIMP-1.

6. Procédé selon la revendication 2, dans lequel ledit deuxième marqueur est la M2-PK.

7. Utilisation de la protéine S100A12 comme molécule marqueur dans le diagnostic du cancer colorectal à partir d'un échantillon de selles obtenu d'un individu.

8. Utilisation de la protéine S100A12 comme molécule marqueur dans le diagnostic précoce du cancer colorectal à partir d'un échantillon de selles obtenu d'un individu.

9. Utilisation de la protéine S100A12 comme molécule marqueur pour le cancer colorectal en combinaison avec une ou plusieurs autres molécule(s) marqueur(s) pour le cancer colorectal choisie(s) dans le groupe constitué par le complexe hémoglobine/haptoglobine, l'hémoglobine, l'inhibiteur tissulaire de métalloprotéases 1 (TIMP-1), et la M2 pyruvate kinase tumorale (M2-PK) dans le diagnostic du cancer colorectal à partir d'un échantillon de selles obtenu d'un individu.
